(19) Europäisches Patentamt
European Patent Office
Office européen des brevets

(11) **EP 4 282 422 A1**

(12) # EUROPEAN PATENT APPLICATION

published in accordance with Art. 153(4) EPC

(43) Date of publication:
**29.11.2023 Bulletin 2023/48**

(21) Application number: **22755761.8**

(22) Date of filing: **11.01.2022**

(51) International Patent Classification (IPC):
**A61K 33/00** (2006.01)     **A61K 9/08** (2006.01)
**A61K 31/045** (2006.01)     **A61P 1/16** (2006.01)

(52) Cooperative Patent Classification (CPC):
**A61K 9/08; A61K 31/045; A61K 33/00; A61P 1/16;
A61P 25/32; A61P 43/00;** Y02E 60/36

(86) International application number:
**PCT/JP2022/000524**

(87) International publication number:
**WO 2022/176435 (25.08.2022 Gazette 2022/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.02.2021  JP 2021024022**

(71) Applicant: **Nihon Trim Co., Ltd.
Osaka 531-0076 (JP)**

(72) Inventor: **HARA Taichi
Tokyo 169-8050 (JP)**

(74) Representative: **Laufhütte, Dieter
Lorenz Seidler Gossel
Rechtsanwälte Patentanwälte
Partnerschaft mbB
Widenmayerstraße 23
80538 München (DE)**

(54) **HYDROGEN WATER FOR PREVENTION OF ALCOHOLIC HEPATOPATHY**

(57)     Hydrogen water for inhibition of alcoholic hepatopathy, to be mixed with ethanol, has a dissolved hydrogen concentration of 550 ppb to 5600 ppb. The concentration of the ethanol in the hydrogen water when mixed with the ethanol is 1% to 4%.

FIG.1

EP 4 282 422 A1

**Description**

TECHNICAL FIELD

The present disclosure relates to hydrogen water for inhibition of alcoholic hepatopathy.

BACKGROUND ART

Various disorders (e.g., ranging from mild disorders such as hangover to severe disorders such as coma and necrosis of stem cells) have been reported for alcoholic hepatopathy, and alcoholic hepatopathy inhibitors containing various active ingredients have been used in light of inhibiting these hepatic disorders.
As the alcoholic hepatopathy inhibitors, there have been proposed, for example, an inhibitor containing, as active ingredients, lysine and citric acid (e.g., see Patent Document 1) and a prevention composition containing proline or lysine (e.g., see Patent Document 2), and an inhibitor containing, as an active ingredient, gelatin or soluble collagen (e.g., see Patent Document 3).

CITATION LIST

PATENT DOCUMENTS

   Patent Document 1: Japanese Unexamined Patent Publication No. 2007-161642
   Patent Document 2: Japanese Unexamined Patent Publication No. H6-116144
   Patent Document 3: Japanese Unexamined Patent Publication No. H6-247876

SUMMARY OF THE INVENTION

TECHNICAL PROBLEM

The conventional alcoholic hepatopathy inhibitors require extraction and synthesis of active ingredients, which complicates the production of the inhibitors and increases its cost.
Hence, the present disclosure was made in view of the above circumstances, and an object of the present disclosure is to provide hydrogen water for inhibition of alcoholic hepatopathy, which is capable of effectively inhibiting alcoholic hepatopathy, highly safe, easy to prepare, and inexpensive.

SOLUTION TO THE PROBLEM

In order to achieve the above objective, the hydrogen water for inhibition of alcoholic hepatopathy according to the present disclosure is to be mixed with ethanol, and has a dissolved hydrogen concentration of 550 ppb to 5600 ppb, and a concentration of the ethanol in the hydrogen water when mixed with the ethanol is 1% to 4%.

ADVANTAGES OF THE INVENTION

According to the present disclosure, hydrogen water for inhibition of alcoholic hepatopathy, which is highly safe, easy to prepare, and inexpensive and capable of effectively inhibiting alcoholic hepatopathy can be provided.

BRIEF DESCRIPTION OF THE DRAWINGS

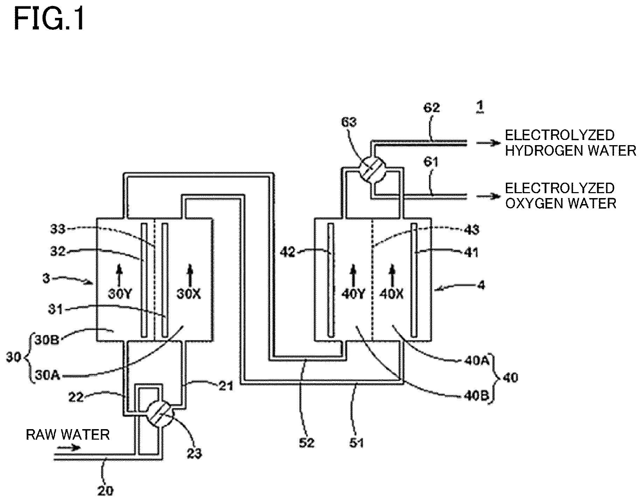

FIG. 1 illustrates a schematic configuration of an electrolyzed water generator configured to generate electrolyzed hydrogen water according to an embodiment of the present disclosure.

DESCRIPTION OF EMBODIMENTS

The hydrogen water of the present disclosure is hydrogen water for inhibition of alcoholic hepatopathy, to be mixed with ethanol, has a dissolved hydrogen concentration of 550 ppb to 5600 ppb, and the concentration of the ethanol in the hydrogen water when mixed with the ethanol is 1% to 4%.
More specifically, it was confirmed in the treatment of model cells (HepG2) derived from human liver cancer with the hydrogen water according to the present disclosure that the cell viability of the cells was improved, and that hepatic cell death was suppressed in the entire range of the dissolved hydrogen concentration of 550 ppb to 5600 ppb as compared with the case where the cells were treated with pure water containing no hydrogen (cell viability: 50%).

This is considered to be because the dissolved hydrogen in the hydrogen water according to the present disclosure reduces the activity of alcohol dehydrogenase (ADH), which is an enzyme which converts ethanol to aldehyde, so that degradation of ethanol into aldehyde in the liver is suppressed, and damage to cells (mitochondrial damage and DNA damage) due to cytotoxicity of aldehyde is reduced, thereby inhibiting alcoholic hepatopathy.

In addition, it is also considered that the dissolved hydrogen in the hydrogen water according to the present disclosure improves the activity of aldehyde dehydrogenase (ALDH), which accelerates the metabolism of aldehydes (oxidation of aldehyde to be degraded into acetic acid) and decreases cell damage (mitochondrial damage and DNA damage) due to the cytotoxicity of aldehyde, thereby inhibiting alcoholic hepatopathy.

When the concentration of the ethanol in the hydrogen water is less than 1%, which is too low, the effect of inhibiting alcoholic hepatopathy may not be sufficiently exhibited.

When the dissolved hydrogen concentration in the hydrogen water is less than 550 ppb, the effect of inhibiting alcoholic hepatopathy may be reduced.

In light of improving the effect of inhibiting alcoholic hepatopathy, the dissolved hydrogen concentration in the hydrogen water is preferably 1100 ppb to 1300 ppb.

As the electrolyzed hydrogen water according to the present disclosure, electrolyzed hydrogen water generated by electrodialysis of water can be used, for example.

FIG. 1 illustrates a schematic configuration of an electrolyzed water generator configured to generate electrolyzed hydrogen water according to the present embodiment.

An electrolyzed water generator 1 includes electrolytic cells 3, 4 and FIG. 1 illustrates the electrolyzed water generator 1 including a pair of electrolytic cells 3, 4. The electrolyzed water generator 1 may include three or more electrolytic cells 3, 4.

The electrolytic cells 3, 4 are connected in series, and the electrolytic cell 3 is provided upstream of the electrolytic cell 4.

The electrolytic cell 3 includes an electrolytic chamber 30 where water is electrolyzed, a first power feeder 31 and a second power feeder 32 arranged opposite to each other in the electrolytic chamber 30, and a membrane 33 which divides the electrolytic chamber 30 into a first pole chamber 30A near the first power feeder 31 and a second pole chamber 30B near the second power feeder 32.

One of the first power feeder 31 or the second power feeder 32 is employed as an anode power feeder, and the other is employed as a cathode power feeder. Water is supplied to both the first pole chamber 30A and the second pole chamber 30B in the electrolytic chamber 30, and a direct-current voltage is applied between the first power feeder 31 and the second power feeder 32, resulting in electrodialysis of water in the electrolytic chamber 30.

An upstream portion of the membrane 33 in the electrolytic chamber 30 is a solid polymer membrane made from a fluorine-based resin having a sulfonate group. A plating layer made from platinum is formed on both surfaces of the membrane 33. As the first power feeder 31 and the second power feeder 32, a platinum plating layer formed on the surface of a reticulated metal such as expanded metal of titanium is employed, for example. Such reticulated first power feeder 31 and second power feeder 32 allow water to spread over the surface of the membrane 33 while sandwiching the membrane 33, thereby promoting electrodialysis in the electrolytic chamber 30.

The plating layer of the membrane 33 is in contact with and electrically connected to the first power feeder 31 and the second power feeder 32. The membrane 33 allows ions generated by electrodialysis to pass therethrough, and the first power feeder 31 and the second power feeder 32 are electrically connected to each other via the membrane 33. In the electrolytic chamber 30 where the membrane 33 made from a solid polymer material is employed, electrodialysis proceeds without increasing the pH value of the electrolyzed hydrogen water (i.e., while water in the electrolytic chamber 30 is maintained neutral).

Water is electrolyzed in the electrolytic chamber 30, thereby generating hydrogen gas and oxygen gas. For example, when the first power feeder 31 is employed as an anode power feeder, oxygen gas and electrolyzed oxygen water dissolving the oxygen gas therein are generated in the first pole chamber 30A. On the other hand, hydrogen gas and electrolyzed hydrogen water dissolving the hydrogen gas therein are generated in the second pole chamber 30B. When the first power feeder 31 is employed as a cathode power feeder, hydrogen gas and electrolyzed hydrogen water dissolving the hydrogen gas therein are generated in the first pole chamber 30A. On the other hand, oxygen gas and electrolyzed oxygen water dissolving the oxygen gas therein are generated in the second pole chamber 30B.

The electrolytic cell 4 includes an electrolytic chamber 40 where water is electrolyzed, a first power feeder 41 and a second power feeder 42 arranged opposite to each other in the electrolytic chamber 40, and a membrane 43 which divides the electrolytic chamber 40 into a first pole chamber 40A near the first power feeder 41 and a second pole chamber 40B near the second power feeder 42.

One of the first power feeder 41 or the second power feeder 42 is employed as an anode power feeder, and the other is employed as a cathode power feeder. Water is supplied to both the first pole chamber 40A and the second pole chamber 40B in the electrolytic chamber 40, and a direct-current voltage is applied between the first power feeder 41 and the second power feeder 42, resulting in electrodialysis of water in the electrolytic chamber 40.

The membrane 43 is, for example, a polytetrafluoroethylene (PTFE) hydrophilic membrane. For example, a metal plate made of titanium or the like is employed as the first power feeder 41 and the second power feeder 42 arranged opposite to each other with the membrane 43 interposed therebetween. The first power feeder 41 and the second power feeder 42 are arranged apart from the membrane 43.

In the electrolytic cell 4 with such a configuration, the electrodialysis proceeds while the pH value of the electrolyzed hydrogen water increases, i.e., while the alkali strength of water in the cathode chamber increases.

When the first power feeder 41 is employed as an anode power feeder, oxygen gas and electrolyzed oxygen water dissolving the oxygen gas therein are generated in the first pole chamber 40A. On the other hand, hydrogen gas and electrolyzed hydrogen water dissolving the hydrogen gas therein are generated in the second pole chamber 40B. When the first power feeder 41 is employed as a cathode power feeder, hydrogen gas and electrolyzed hydrogen water dissolving the hydrogen gas therein are generated in the first pole chamber 40A. On the other hand, oxygen gas and electrolyzed oxygen water dissolving the oxygen gas therein are generated in the second pole chamber 40B.

The electrolyzed water generator 1 further includes a water supply path 20 for supplying water to be electrolyzed in the electrolytic chambers 30, 40, and water release paths 61, 62 for releasing electrolyzed water from the electrolytic chambers 30, 40.

Raw water is supplied from the water supply path 20 into the electrolyzed water generator 1. Tap water is generally used as raw water, but other water such as well water and ground water may also be used. For example, when the electrolyzed water generator 1 is used for generation of drinking electrolyzed hydrogen water, the water supply path 20 is appropriately provided with a water purification cartridge for purifying raw water.

The water supply path 20 is branched into a water supply path 21 and a water supply path 22. The water supply path 21 is in connection with a lower stage portion of the first pole chamber 30A. The water supply path 22 is in connection with a lower end portion of the second pole chamber 30B. Water flowing into the water supply path 20 passes through the water supply paths 21, 22, and flows into the first pole chamber 30A and the second pole chamber 30B.

The pathway of the water supply paths 21, 22 is provided with a flow regulating valve 23, and the flow regulating valve 23 regulates the amount of water flowing through the water supply paths 21, 22. Hence, the flow regulating valve 23 regulates the amount of water flowing into the first pole chamber 30A and the second pole chamber 30B.

The water release path 61 is in connection with an upper end portion of the first pole chamber 40A, which allows water flowing out of the first pole chamber 40A to flow into the water release path 61. The water release path 62 is in connection with an upper end portion of the second pole chamber 40B, which allows water flowing out of the second pole chamber 40B to flow into the water release path 62.

A flow path switching valve 63 is provided between the first and second pole chambers 40A, 40B and the water release paths 61, 62, and selectively switches the connection between the first and second pole chambers 40A, 40B and the water release paths 61, 62.

Electrolytic currents supplied to the power feeders 31, 32 and the power feeders 41, 42 are controlled by a controller (not shown). The controller controls each unit, such as the power feeders 31, 32 and the power feeders 41, 42. The controller includes, for example, a central processing unit (CPU) that executes various types of arithmetic processing, information processing, and the like, and a memory that stores a program that controls the operation of the CPU and various types of information. For example, the controller controls polarities of the first power feeders 31, 41 and the second power feeders 32, 42.

The polarities of the first power feeders 31, 41 and the second power feeders 32, 42 are mutually changed to allow desired electrolyzed water out of the electrolyzed hydrogen water and the electrolyzed oxygen water to be released through the water release path 61 and unnecessary electrolyzed water to be released through the water release path 62. In addition, the time during which the first power feeders 31, 41 and the second power feeders 32, 42 function as the anode power feeders or the cathode power feeders is equalized, and the adhesion of scales in the electrolytic chamber 30 and the electrolytic chamber 40 can thus be suppressed.

The first pole chamber 30A of the electrolytic chamber 30 and the first pole chamber 40A of the electrolytic chamber 40 communicate with each other in series by a first water path 51. The second pole chamber 30B of the electrolytic chamber 30 and the second pole chamber 40B of the electrolytic chamber 40 communicate with each other in series by a second water path 52.

In the embodiment described above, the hydrogen water for inhibition of alcoholic hepatopathy, to be mixed with ethanol, is used; however, for example, a hydrogen-containing solution dissolving hydrogen in an ethanol solution by direct contact between the ethanol solution and hydrogen gas may also be used.

More specifically, a method can be employed in which a membrane module including a sleeve into which hydrogen gas is supplied and hollow fibers disposed inside the sleeve and having multiple pores is used to bring hydrogen gas into direct contact with an ethanol solution by bubbling via the pores in the hollow fibers, thereby generating an ethanol solution dissolving hydrogen therein.

Also in such a case, the concentration of the ethanol is set to 1% to 4%, and the dissolved hydrogen concentration is set to 550 ppb to 5600 ppb. This makes it possible to obtain the same effect as the hydrogen water.

[Example]

The present disclosure will be described below based on the Examples. The present disclosure is not limited to these examples, and these examples can be modified and changed based on the intent of the present disclosure. Such modifications and changes are not excluded from the scope of the present disclosure.

<Production of Electrolyzed Hydrogen Water>

Electrolyzed hydrogen water of different dissolved hydrogen concentrations of levels 1 to 4 was obtained by using an electrolyzed water generator (manufactured by Nihon Trim Co., Ltd., trade name: TRIMIONGRACE) under conditions of a temperature of 22°C and a flow rate of 1.5 L/min, and pure water was obtained through filtration with micro carbon.

Next, the pH and the dissolved hydrogen concentration of the electrolyzed hydrogen water of each level were measured. For the measurement of pH, a pH meter (manufactured by HORIBA, Ltd., trade name: LAQUAactD-71) was used. For the measurement of the dissolved hydrogen concentration, a dissolved hydrogen meter DH-35A (manufactured by DKK-TOA CORPORATION) was used.

Level 1: 800 ppb, pH 7
Level 2: 860 ppb, pH 8
Level 3: 1120 ppb, pH 9
Level 4: 1320 ppb, pH 10

<Production of Medium for Treating Electrolyzed Hydrogen Water (Electrolyzed Hydrogen Water Medium)>

Each of 5 × Dulbecco's modified Eagle's media (DMEMs) prepared from powder was diluted 5-fold with the pure water described above or electrolyzed hydrogen water of each level, and used as a medium for treating electrolyzed hydrogen water (i.e., treatment media which are hydrogen water mixtures obtained by mixing 20% 5 × DMEMs with 80% electrolyzed hydrogen water or pure water).

The dissolved hydrogen concentration of the electrolyzed hydrogen water medium of each level was as follows.

Level 1: 800 ppb × 4/5 = 640 ppb
Level 2: 860 ppb × 4/5 = 688 ppb
Level 3: 1120 ppb × 4/5 = 896 ppb
Level 4: 1320 ppb × 4/5 = 1056 ppb

<Cell Culturing>

AHepG2 cell line, which is human liver cancer-derived cells, was obtained from Riken Bioresource Research Center (RIKEN BRC) and cultured in DMEM containing 10% fetal bovine serum (FBS) (Sigma-Aldrich, F7524) and 1% penicillin-streptomycin (manufactured by FUJIFILM Wako Pure Chemical Corporation, trade name: 168-23191) under conditions of 37°C and 5% $CO_2$.

<Evaluation of Ethanol Toxicity to Liver Cells>

The HepG2 cells were seeded at $5.0 \times 10^4$ cells/24-well plate, pre-cultured for 24 hours, and treated with 0% to 8% ethanol for 24 hours. Thereafter, the cells were detached and suspended using trypsin, the viable cell count was determined using a staining method (trypan blue method) with trypan blue (manufactured by FUJIFILM Wako Pure Chemical Corporation, trade name: 207-17081), and the cell viability of the HepG2 cells after the ethanol treatment was calculated using the following equation (1). Table 1 shows the results.

[Mathematical 1]

$$\text{Cell viability} = (\text{viable cell count at each ethanol concentration}/\text{viable cell count at 0\%}$$

$$\text{ethanol concentration}) \times 100 \quad (1)$$

[Table 1]

|  | Ethanol concentration | | | | | | |
|---|---|---|---|---|---|---|---|
|  | 0 | 0.25 | 0.5 | 1 | 2 | 4 | 8 |
| Viable cell count [$\times 10^4$ cells/well] | 9.80 | 9.48 | 9.37 | 8.22 | 6.05 | 4.87 | 0.35 |
| Cell viability [%] | 100 | 96.7 | 95.6 | 83.8 | 61.7 | 49.7 | 3.6 |

As can be seen from Table 1, as the ethanol concentration increases, the cell viability decreases, indicating that ethanol is toxic to liver cells.

<Evaluation of Action of Electrolyzed Hydrogen Water on Ethanol Toxicity>

HepG2 cells were seeded at $5.0 \times 10^4$ cells/24-well plate, pre-cultured for 24 hours, and treated for 24 hours with pure water, the electrolyzed hydrogen water media of levels 1 to 4, pure water containing 4% ethanol, and the electrolyzed hydrogen water media of levels 1 to 4 containing 4% ethanol. Thereafter, the cells were detached and suspended using trypsin, the viable cell count was determined using a staining method (trypan blue method) with trypan blue (manufactured by FUJIFILM Wako Pure Chemical Corporation, trade name: 207-17081), and the cell viability of the HepG2 cells after the ethanol treatment was calculated using the following equation (2). Tables 2 and 3 show the results.
[Mathematical 2]

$$\text{Cell viability} = (\text{viable cell count in each ethanol treated group}/\text{viable cell count in}$$

$$\text{each ethanol untreated group}) \times 100 \quad (2)$$

[Table 2]

|  | No ethanol | | | | | Containing 4% ethanol | | | | |
|---|---|---|---|---|---|---|---|---|---|---|
|  | Pure water | LV of electrolyzed hydrogen water | | | | Pure water | LV of electrolyzed hydrogen water | | | |
|  |  | LV1 | LV2 | LV3 | LV4 |  | LV1 | LV2 | LV3 | LV4 |
| Viable cell count [$\times 10^4$ cells/well] | 10.30 | 10.02 | 9.85 | 9.70 | 9.38 | 5.47 | 5.73 | 5.80 | 5.82 | 6.20 |

[Table 3]

|  | No ethanol | | | | | Containing 4% ethanol | | | | |
|---|---|---|---|---|---|---|---|---|---|---|
|  | Pure , water | LV of electrolyzed hydrogen water | | | | Pure water | LV of electrolyzed hydrogen water | | | |
|  |  | LV1 | LV2 | LV3 | LV4 |  | LV1 | LV2 | LV3 | LV4 |
| Cell viability [%] | 100.0 | 100.0 | 100.0 | 100.0 | 100.0 | 53.1 | 57.2 | 58.9 | 60.0 | 66.1 |

As can be seen from Table 3, the treatments with the electrolyzed hydrogen water media of levels 1 to 4 containing 4% ethanol showed a higher cell viability than that in the treatment with pure water containing 4% ethanol (containing no electrolyzed hydrogen water). This indicates that the electrolyzed hydrogen water of levels 1 to 4 has the effect of reducing the toxicity of ethanol to liver cells.

<Evaluation of Action of Electrolyzed Hydrogen Water at each Ethanol Concentration>

HepG2 cells were seeded at $5.0 \times 10^4$ cells/24-well plate, pre-cultured for 24 hours, and treated for 24 hours with pure water containing 0% to 8% ethanol and the electrolyzed hydrogen water medium of level 4 containing 0% to 8% ethanol. Thereafter, the cells were detached and suspended using trypsin, the viable cell count was determined using a staining method (trypan blue method) with trypan blue (manufactured by FUJIFILM Wako Pure Chemical Corporation, trade name: 207-17081), and the cell viability of the HepG2 cells after the ethanol treatment was calculated using the following equation (3). Tables 4 and 5 show the results.
[Mathematical 3]

$$\text{Cell viability} = (\text{viable cell count at each ethanol concentration/viable cell count at 0\%}$$

$$\text{ethanol concentration}) \times 100 \quad (3)$$

[Table 4]

| | Pure water | | | | | Electrolyzed hydrogen water of LV4 | | | | |
|---|---|---|---|---|---|---|---|---|---|---|
| | Ethanol content [%] | | | | | Ethanol content [%] | | | | |
| | 0 | 1 | 2 | 4 | 8 | 0 | 1 | 2 | 4 | 8 |
| Viable cell count [$\times 10^4$ cells/well] | 10.27 | 8.58 | 6.28 | 4.80 | 0.23 | 9.37 | 8.50 | 6.50 | 5.35 | 0.15 |

[Table 5]

| | Pure water | | | | | Electrolyzed hydrogen water of LV4 | | | | |
|---|---|---|---|---|---|---|---|---|---|---|
| | Ethanol content [%] | | | | | Ethanol content [%] | | | | |
| | 0 | 1 | 2 | 4 | 8 | 0 | 1 | 2 | 4 | 8 |
| Cell viability [%] | 100.0 | 83.5 | 61.1 | 46.7 | 2.2 | 100.0 | 90.7 | 69.4 | 57.1 | 1.6 |

As can be seen from Table 5, the treatments with the electrolyzed hydrogen water medium of level 4 containing 1% to 4% ethanol showed a higher cell viability than that in the treatment with pure water containing 1% to 4% ethanol (containing no electrolyzed hydrogen water). This indicates that the electrolyzed hydrogen water of level 4 containing 1% to 4% ethanol has the effect of reducing the toxicity of ethanol to liver cells, and in particular, the electrolyzed hydrogen water of level 4 containing 2% to 4% ethanol has the effect of dramatically reducing the toxicity of ethanol to liver cells.

<Evaluation of Action of Dissolved Hydrogen on Ethanol Toxicity>

HepG2 cells were seeded at $5.0 \times 10^4$ cells/24-well plate, pre-cultured for 24 hours, and then treated for 24 hours with pure water, an electrolyzed hydrogen water medium of level 4, an electrolyzed hydrogen water medium of level 4 (AC) which had been autoclaved (AC) twice, an electrolyzed hydrogen water medium of level 4 (pH) which had been pH-adjusted (neutralized) with a buffer, a hydrogen water medium having a dissolved hydrogen concentration of 1040 ppb (5 $\times$ Dulbecco-modified Eagles media (DMEMs) diluted five-fold with hydrogen water having a dissolved hydrogen concentration of 1300 ppb), and each water and each medium containing 4% ethanol. Thereafter, the cells were detached and suspended using trypsin, the viable cell count was determined using a staining method (trypan blue method) with trypan blue (manufactured by FUJIFII,M Wako Pure Chemical Corporation, trade name: 207-17081), and the cell viability of the HepG2 cells after the ethanol treatment was calculated using the above equation (2). Tables 6 and 7 show the results.

[Table 6]

| | No ethanol | | | | | Containing 4% ethanol | | | | |
| --- | --- | --- | --- | --- | --- | --- | --- | --- | --- | --- |
| | Pure water | Electrolyzed hydrogen water | | | Hydrogen water | Pure water | Electrolyzed hydrogen water | | | Hydrogen water |
| | | LV4 | LV4 (AC) | LV4 (pH) | | | LV4 | LV4 (AC) | LV4 (pH) | |
| Viable cell count [$\times 10^4$ cells/ well] | 10.18 | 9.40 | 10.05 | 9.62 | 9.82 | 4.83 | 5.60 | 4.88 | 6.12 | 5.72 |

[Table 7]

| | No ethanol | | | | | Containing 4% ethanol | | | | |
| --- | --- | --- | --- | --- | --- | --- | --- | --- | --- | --- |
| | Pure water | Electrolyzed hydrogen water | | | Hydrogen water | Pure water | Electrolyzed hydrogen water | | | Hydrogen water |
| | | LV4 | LV4 (AC) | LV4 (pH) | | | LV4 | LV4 (AC) | LV4 (pH) | |
| Cell viability [%] | 100.0 | 100.0 | 100.0 | 100.0 | 100.0 | 47.4 | 59.6 | 48.6 | 63.6 | 58.2 |

As can be seen from Table 7, the treatment with the electrolyzed hydrogen water medium of level 4 (AC) which contained 4% ethanol and had been autoclaved twice showed a comparable cell viability to that of the treatment with pure water containing 4% ethanol (containing no electrolyzed hydrogen water). This indicates that the electrolyzed hydrogen water, from which dissolved hydrogen has been removed by autoclaving, has no effect of reducing the toxicity of ethanol to liver cells. That is, it is demonstrated that hydrogen in the electrolyzed hydrogen water has the effect of reducing the toxicity of ethanol to liver cells.

Furter, the treatment with the electrolyzed hydrogen water medium of level 4 (pH) which contained 4% ethanol and had been pH-adjusted (neutralized) with a buffer (HEPES) shows a comparable cell viability to that of the treatment with the electrolyzed hydrogen water medium of level 4 containing 4% ethanol. This indicates that there is no influence of the pH adjustment of the electrolyzed hydrogen water.

Further, the treatment with the hydrogen water medium having a dissolved hydrogen concentration of 1040 ppb shows a comparable cell viability to that of the treatment with the electrolyzed hydrogen water medium of level 4 containing 4% ethanol. This indicates that the hydrogen water which is not generated by electrolysis has the effect of reducing the toxicity of ethanol to liver cells as in the electrolyzed hydrogen water.

<Evaluation of Action of Hydrogen Water on Ethanol Concentration in Cell Culture Medium>

HepG2 cells were seeded at $2.0 \times 10^5$ cells/6-well plate, pre-cultured for 24 hours, and then treated for 24 hours with pure water containing 4% ethanol, an electrolyzed hydrogen water medium of level 4 containing 4% ethanol, an electrolyzed hydrogen water medium of level 4 (AC) which contained 4% ethanol and had been autoclaved (AC) twice, a hydrogen water medium containing 4% ethanol and having a dissolved hydrogen concentration of 1040 ppb (5 $\times$ Dulbecco-modified Eagles media (DMEMs) diluted five-fold with hydrogen water having a dissolved hydrogen concentration of 1300 ppb).

Thereafter, the ethanol concentration [%] in each culture supernatant was measured using a QuantiChrom™ Ethanol Assay Kit. Table 8 shows the results.

[Table 8]

| | Containing 4% ethanol | | | |
|---|---|---|---|---|
| | Pure water | Electrolyzed hydrogen water | | Hydrogen water |
| | | LV4 | LV4 (AC) | |
| Ethanol concentration [%] | 1.77 | 2.21 | 1.76 | 2.21 |

As can be seen from Table 8, the treatments with the electrolyzed hydrogen water medium of level 4 and the hydrogen water medium showed a higher ethanol concentration in the cell culture solution than those in the treatments with pure water and the electrolyzed hydrogen water medium of level 4 (AC) which had been autoclaved twice.

This is considered to be because the dissolved hydrogen in the electrolyzed hydrogen water medium of level 4 and the hydrogen water medium decreased the activity of alcohol dehydrogenase (ADH), thereby inhibiting the metabolism of ethanol (oxidization of ethanol into aldehyde).

<Evaluation of Action of Hydrogen Water on Aldehyde Concentration in Cells>

HepG2 cells were seeded at $1.0 \times 10^6$ cells/10-cm dish, pre-cultured for 24 hours, and then treated for 24 hours with pure water containing 4% ethanol, an electrolyzed hydrogen water medium of level 4 containing 4% ethanol, an electrolyzed hydrogen water medium of level 4 (AC) which contained 4% ethanol and had been autoclaved (AC) twice, a hydrogen water medium containing 4% ethanol and having a dissolved hydrogen concentration of 1040 ppb (5 $\times$ Dulbecco-modified Eagles media (DMEMs) diluted five-fold with hydrogen water having a dissolved hydrogen concentration of 1300 ppb).

Thereafter, the acetaldehyde concentration [nmol] in $1.0 \times 10^6$ cells was measured in the supernatant of the cultured cells dissolved in Lysis bf using an EnzyChrom™ Acetaldehyde Assay Kit. Table 9 shows the results.

[Table 9]

| | Containing 4% ethanol | | | |
|---|---|---|---|---|
| | Pure water | Electrolyzed hydrogen water | | Hydrogen water |
| | | LV4 | LV4 (AC) | |
| Aldehyde concentration [nmol] | 5.86 | 4.38 | 5.61 | 4.29 |

As can be seen from Table 9, the treatments with the electrolyzed hydrogen water medium of level 4 and the hydrogen water medium showed a higher aldehyde concentration in the cells than those in the treatments with pure water and the electrolyzed hydrogen water medium of level 4 (AC) which had been autoclaved twice.

This is considered to be because the dissolved hydrogen in the electrolyzed hydrogen water medium of level 4 and the hydrogen water medium increased the activity of aldehyde dehydrogenase (ALDH), thereby promoting the metabolism of aldehyde (oxidization of aldehyde into acetic acid).

<Evaluation of Action of Dissolved Hydrogen on Alcohol Dehydrogenase (ADH)>

HepG2 cells were seeded at $2.0 \times 10^5$ cells/6-well plate, pre-cultured for 24 hours, and then treated for 24 hours with pure water, an electrolyzed hydrogen water medium of level 4, an electrolyzed hydrogen water medium of level 4 (AC) which had been autoclaved (AC) twice, a hydrogen water medium having a dissolved hydrogen concentration of 1040 ppb (5 $\times$ Dulbecco-modified Eagles media (DMEMs) diluted five-fold with hydrogen water having a dissolved hydrogen concentration of 1300 ppb), and each water and each medium containing 4% ethanol.

Thereafter, the ADH activity [μU] in the $1.0 \times 10^6$ cells was measured using an Alcohol Dehydrogenase Activity Colorimetric Assay Kit. Table 10 shows the results.

[Table 10]

| | No ethanol | | | | Containing 4% ethanol | | | |
|---|---|---|---|---|---|---|---|---|
| | Pure water | Electrolyzed hydrogen water | | Hydrogen water | Pure water | Electrolyzed hydrogen water | | Hydrogen water |
| | | LV4 | LV4 (AC) | | | LV4 | LV4 (AC) | |
| ADH activity [μU] | 332.0 | 343.1 | 333.7 | 342.5 | 368.3 | 289.1 | 359.3 | 271.6 |

As can be seen from Table 10, the treatment with the electrolyzed hydrogen water medium of level 4 containing 4% ethanol showed a lower ADH activity than that in the treatment with the electrolyzed hydrogen water medium of level 4 containing no ethanol. Similarly, the treatment with the hydrogen water medium containing 4% ethanol showed a lower ADH activity than that in the treatment with the hydrogen water medium containing no ethanol.

<Evaluation of Action of Dissolved Hydrogen on Aldehyde Dehydrogenase (ALDH)>

HepG2 cells were seeded at $2.0 \times 10^5$ cells/6-well plate, pre-cultured for 24 hours, and then treated for 24 hours with pure water, an electrolyzed hydrogen water medium of level 4, an electrolyzed hydrogen water medium of level 4 (AC) which had been autoclaved (AC) twice, a hydrogen water medium having a dissolved hydrogen concentration of 1040 ppb ($5 \times$ Dulbecco-modified Eagles media (DMEMs) diluted five-fold with hydrogen water having a dissolved hydrogen concentration of 1300 ppb), and each water and each medium containing 4% ethanol.
Thereafter, the ALDH activity [μU] in the $1.0 \times 10^6$ cells was measured using an Aldehyde Dehydrogenase Activity Colorimetric Assay Kit. Table 11 shows the results.

[Table 11]

| | No ethanol | | | | Containing 4% ethanol | | | |
|---|---|---|---|---|---|---|---|---|
| | Pure water | Electrolyzed hydrogen water | | Hydrogen water | Pure water | Electrolyzed hydrogen water | | Hydrogen water |
| | | LV4 | LV4 (AC) | | | LV4 | LV4 (AC) | |
| ALDH activity [μU] | 271.0 | 338.4 | 279.5 | 338.7 | 273.5 | 343.1 | 278.8 | 326.8 |

As can be seen from Table 11, the treatment with the electrolyzed hydrogen water medium of level 4 showed higher ALDH activity than that in the treatment with pure water regardless of the presence or absence of ethanol. Similarly, the treatment with the hydrogen water medium showed a higher ALDH activity than that in the treatment with pure water regardless of the presence or absence of ethanol.

<Evaluation of Action of Dissolved Hydrogen on Aldehyde Toxicity>

HepG2 cells were seeded at $5.0 \times 10^4$ cells/24-well plate, pre-cultured for 24 hours, and then treated for 24 hours with pure water, an electrolyzed hydrogen water medium of level 4, an electrolyzed hydrogen water medium of level 4 (AC) which had been autoclaved (AC) twice, a hydrogen water medium having a dissolved hydrogen concentration of 1040 ppb ($5 \times$ Dulbecco-modified Eagles media (DMEMs) diluted five-fold with hydrogen water having a dissolved hydrogen concentration of 1300 ppb), and each water and each medium containing 1 mM acetaldehyde. Thereafter, the cells were detached and suspended using trypsin, the viable cell count was determined using a staining method (trypan blue method) with trypan blue (manufactured by FUJIFILM Wako Pure Chemical Corporation, trade name: 207-17081), and the cell viability of the HepG2 cells after the aldehyde treatment was calculated using the following equation (4). Tables 12 and 13 show the results.
[Mathematical 4]

$$\text{Cell viability} = (\text{viable cell count in each aldehyde treated group/viable cell count in each aldehyde untreated group}) \times 100 \quad (4)$$

[Table 12]

| | No ethanol | | | | Containing 1mM aldehyde | | | |
|---|---|---|---|---|---|---|---|---|
| | Pure water | Electrolyzed hydrogen water | | Hydrogen water | Pure water | Electrolyzed hydrogen water | | Hydrogen water |
| | | LV4 | LV4 (AC) | | | LV4 | LV4 (AC) | |
| Viable cell count [$\times 10^4$ cells/well] | 10.30 | 9.75 | 10.15 | 10.23 | 6.28 | 7.20 | 6.22 | 7.47 |

[Table 13]

| | No ethanol | | | | Containing 1mM aldehyde | | | |
|---|---|---|---|---|---|---|---|---|
| | Pure water | Electrolyzed hydrogen water | | Hydrogen water | Pure water | Electrolyzed hydrogen water | | Hydrogen water |
| | | LV4 | LV4 (AC) | | | LV4 | LV4 (AC) | |
| Cell viability [%] | 100.0 | 100.0 | 100.0 | 100.0 | 61.0 | 73.8 | 61.3 | 73.0 |

As can be seen from Table 13, the treatment with the electrolyzed hydrogen water medium of level 4 containing 1 mM aldehyde showed a higher cell viability than that in the treatment with pure water containing 1 mM aldehyde. This indicates that the electrolyzed hydrogen water of level 4 has the effect of reducing toxicity of aldehyde to liver cells.

Similarly, the treatment with the hydrogen water medium containing 1 mM aldehyde showed a higher cell viability than that in the treatment with pure water containing 1 mM aldehyde. This indicates that the hydrogen water has the effect of reducing toxicity of aldehyde to liver cells.

<Evaluation of Action of Hydrogen Water on Ethanol Toxicity>

A hydrogen water generation kit (manufactured by Nihon Trim Co., Ltd., trade name: TRIM SEVEN WATER) was used to generate hydrogen water of 7000 ppb. Next, the hydrogen generation agent supplied with the kit was soaked in pure water for 5 seconds, placed in a special capsule supplied with the kit, and then placed in a PET bottle supplied with the kit filled with pure water, tightly closed, and allowed to stand for 5 minutes. The PET bottle was then shaken for 30 seconds and allowed to stand at 4°C for 24 hours. The PET bottle was then further shaken for 30 seconds. Thus, hydrogen water of 7000 ppb was obtained. Hydrogen water of 1300 ppb was obtained by diluting the hydrogen water of 7000 ppb with pure water.

Next, HepG2 cells were seeded at $5.0 \times 10^4$ cells/24-well plate, pre-cultured for 24 hours, and then treated for 24 hours with pure water, a hydrogen water medium having a dissolved hydrogen concentration of 1040 ppb, a hydrogen water medium having a dissolved hydrogen concentration of 5600 ppb ($5 \times$ Dulbecco-modified Eagles media (DMEMs) diluted five-fold with hydrogen water having a dissolved hydrogen concentration of 1300 ppb or 7000 ppb), and each water and each medium containing 4% ethanol. Thereafter, the cells were detached and suspended using trypsin, the viable cell count was determined using a staining method (trypan blue method) with trypan blue (manufactured by FUJIFILM Wako Pure Chemical Corporation, trade name: 207-17081), and the cell viability of the HepG2 cells after the ethanol treatment was calculated using the above equation (2). Tables 14 and 15 show the results.

[Table 14]

| | No ethanol | | | Containing 4% ethanol | | |
|---|---|---|---|---|---|---|
| | Pure water | Hydrogen water | | Pure water | Hydrogen water | |
| | | 1040 ppb | 5600 ppb | | 1040 ppb | 5600 ppb |
| Viable cell count [× $10^4$ cells/well] | 10.05 | 9.62 | 9.32 | 4.77 | 5.32 | 5.55 |

[Table 15]

| | No ethanol | | | Containing 4% ethanol | | |
|---|---|---|---|---|---|---|
| | Pure water | Hydrogen water | | Pure water | Hydrogen water | |
| | | 1040 ppb | 5600 ppb | | 1040 ppb | 5600 ppb |
| Cell viability [%] | 100.0 | 100.0 | 100.0 | 45.6 | 55.3 | 59.6 |

As can be seen from Table 15, the treatments with the electrolyzed hydrogen water medium of 1040 ppb containing 4% ethanol and the electrolyzed hydrogen water medium of 5600 ppb containing 4% ethanol showed a higher cell viability than that in the treatment with pure water containing 4% ethanol (containing no hydrogen water). This indicates that the electrolyzed hydrogen water has the effect of reducing the toxicity of ethanol to liver cells.

DESCRIPTION OF REFERENCE CHARACTERS

| | |
|---|---|
| 1 | Electrolyzed Water Generator |
| 3 | Electrolytic Cell |
| 4 | Electrolytic Cell |
| 20 to 22 | Water Supply Path |
| 30 | Electrolytic Chamber |
| 30A | First Pole Chamber |
| 30B | Second Pole Chamber |
| 31 | First Power Feeder |
| 32 | Second Power Feeder |
| 33 | Membrane |
| 40 | Electrolytic Chamber |
| 40A | First Pole Chamber |
| 40B | Second Pole Chamber |
| 41 | First Power Feeder |
| 42 | Second Power Feeder |
| 43 | Membrane |
| 61 | Water Release Path |
| 62 | Water Release Path |

**Claims**

1. Hydrogen water for inhibition of alcoholic hepatopathy, to be mixed with ethanol, the hydrogen water having:

    a dissolved hydrogen concentration of 550 ppb to 5600 ppb, and
    a concentration of the ethanol in the hydrogen water when mixed with the ethanol being 1% to 4%.

2. The hydrogen water of claim 1, wherein the dissolved hydrogen concentration is 1100 ppb to 1300 ppb.

3. The hydrogen water of claim 1 or 2, wherein the hydrogen water is electrolyzed hydrogen water.

4. A method for treating ethanol, the method comprising mixing hydrogen water for inhibition of alcoholic hepatopathy having a dissolved hydrogen concentration of 550 ppb to 5600 ppb with ethanol so as to have a concentration of the ethanol in the hydrogen water of 1% to 4%.

# FIG.1

**INTERNATIONAL SEARCH REPORT**

| International application No. |
| --- |
| **PCT/JP2022/000524** |

| A. | CLASSIFICATION OF SUBJECT MATTER |
| --- | --- |

*A61K 33/00*(2006.01)i; *A61K 9/08*(2006.01)i; *A61K 31/045*(2006.01)i; *A61P 1/16*(2006.01)i
FI: A61K33/00; A61P1/16; A61K9/08; A61K31/045

According to International Patent Classification (IPC) or to both national classification and IPC

| B. | FIELDS SEARCHED |
| --- | --- |

Minimum documentation searched (classification system followed by classification symbols)

A61K33/00; A61K9/08; A61K31/045; A61P1/16

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CHING-PIN, Lin et al. Anti-oxidant and anti-inflammatory effects of hydrogenrich water alleviate ethanol-induced fatty liver in mice. World J. Gastroenterol, 2017, vol. 23, pp. 4920-4934 <br> abstract | 1-4 |
| Y | 二日酔いには水素が効く？, 三旺コーポレーション [online], 2020, internet: <URL: https://sanoh-corp.jp/post-851/>, [retrieved on 01 February 2022] non-official translation (Does hydrogen work on hangovers. Sanoh Corp.) <br> entire text, paragraphs 6, 7 | 1-4 |
| Y | Lets enjoy alcohol with electrolytes! お酒を飲むときの電解水素水の使い方, 水と健康 の情悤メディア・トリム・ミズラボ [online], 2019, <URL:https://www.nihon-trim.co.jp/ media/13652/>, [retrieved on 01 January] non-official translation (How to use electrolytic water when drinking alchohol. Water and health information media: Trim Mizu-labo.) <br> entire text, paragraphs 4, 8-11 | 1-4 |
| Y | JP 2005-192542 A (FUJISHITA, Keisuke) 21 July 2005 (2005-07-21) <br> claim 1 | 1-4 |

☑ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 February 2022** | **15 February 2022** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

International application No.

**PCT/JP2022/000524**

| C. | DOCUMENTS CONSIDERED TO BE RELEVANT | |
|---|---|---|
| Category* | Citation of document, with indication, where appropriate, of the relevant passages | Relevant to claim No. |
| Y | JP 2015-78140 A (SHIZUOKA PREFECTURAL UNIVERSITY CORP.) 23 April 2015 (2015-04-23)<br>claim 6, paragraph [0042] | 1-4 |
| Y | KATO, Shinya et al. The hydrogen-storing microporous silica Microcluster reduces acetaldehyde contained in a distilled spirit. Materials Science and Engineering C, 2016, vol. 69, pp. 117-121<br>abstract, conclusion | 1-4 |

**INTERNATIONAL SEARCH REPORT**
Information on patent family members

International application No.

**PCT/JP2022/000524**

| Patent document cited in search report | | | Publication date (day/month/year) | Patent family member(s) | Publication date (day/month/year) |
|---|---|---|---|---|---|
| JP | 2005-192542 | A | 21 July 2005 | (Family: none) | |
| JP | 2015-78140 | A | 23 April 2015 | (Family: none) | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007161642 A
- JP H6116144 A
- JP H6247876 A